# EUROPEAN PATENT SPECIFICATION

(11) **EP 4 528 745 B1**
(45) Date of publication and mention of the grant of the patent: **21.10.2026**
(21) Application number: 25156053.8
(22) Date of filing: 30.12.2021
(51) Int. Cl.: A61M 5/142, G16H 20/17

(54) **SYSTEMS AND METHODS FOR OCCLUSION DETECTION IN INFUSION PUMPS**
SYSTEME UND VERFAHREN ZUR OKKLUSIONSERKENNUNG IN INFUSIONSPUMPEN
SYSTÈMES ET PROCÉDÉS DE DÉTECTION D'OCCLUSION DANS DES POMPES À PERFUSION

(30) Priority: 30.12.2020 US 202063132183 P
(43) Date of publication of application: 26.03.2025
(62) Document number(s) of the earlier application(s) in accordance with Art. 76 EPC: 21840386.3 / 4 271 444
(73) Proprietor: BAXTER INTERNATIONAL INC., Deerfield, IL 60015 (US); BAXTER HEALTHCARE SA, 8152 Glattpark (Opfikon) (CH)
(72) Inventor: FISCHER, Steven Ward, Deerfield, 60015 (US); CHEN, Ye, Deerfield, 60015 (US)
(74) Representative: Potter Clarkson

(56) References cited:
- US-A1- 2013 253 420
- US-A1- 2014 358 077
- US-A1- 2019 117 891

## Description

### BACKGROUND

Generally, medical patients sometimes require precise intravenous ("IV") delivery of either continuous medication or medication at set periodic intervals using infusion pumps. Known infusion pumps provide controlled fluid medication or drug infusion where the fluid can be administered at a precise rate that keeps a medication/drug concentration within a therapeutic margin and out of an unnecessary or possibly toxic range. The infusion pumps provide appropriate medication/drug delivery to a patient at a controllable rate, which does not require frequent attention.

Infusion pumps may facilitate administration of intravenous therapy to patients both in and outside of a clinical setting. Outside a clinical setting, doctors have found that in many instances patients can return to substantially normal lives, provided that they receive periodic or continuous intravenous administration of medication, drugs, or other fluids such as saline. Among the types of therapies requiring this kind of administration are antibiotic therapy, chemotherapy, pain control therapy, nutritional therapy, and several other types that are known by those skilled in the art. In many cases, patients receive multiple daily therapies. Certain medical conditions require infusion of drugs in a solution over relatively short periods such as from thirty minutes to two hours. These conditions and others have collectively promoted the development of increasingly lightweight, portable or ambulatory infusion pumps that can be worn by a patient and are capable of administering a continuous supply of medication at a desired rate, or providing several doses of medication at scheduled intervals.

Known infusion pumps include elastomeric pumps, which squeeze solution from flexible containers, such as balloons, into IV tubing for delivery to a patient. Alternatively, infusion pumps may include spring-loaded pumps that pressurize solution containers or reservoirs. Certain pump designs utilize cartridges containing flexible compartments that are squeezed by pressure rollers for discharging the solutions. Further, known infusion pumps include peristaltic pumps having finger actuators or a roller actuator that apply pressure to IV tubing for delivering fluid from a fluid container to a patient.

Infusion pumps utilizing syringes are also known. These syringe pumps use a drive mechanism to move a plunger of a syringe to deliver a fluid to a patient. Typically, these infusion pumps include a housing adapted to receive a syringe assembly, a drive mechanism adapted to move the syringe plunger, and a pump control unit having a variety of operating controls.

In order to ensure a safe and effective operation of an infusion system, infusion pumps may need to be monitored for risk of an occlusion. An occlusion involves any blockage or a closing of IV tubing connected to an infusion pump, which may thereby restrict the flow of fluid to a patient. An occlusion can occur for a variety of reasons, including, but not limited to, unintended pressure applied to a pump, a bending of IV tubing, and/or clogging caused by particles within a fluid.

Conventional methods for occlusion detection use a force sensor to detect an in-line IV tubing pressure. These force sensor measurements indirectly indicate an occlusion when the in-line pressure reaches or exceeds a threshold. For example, IV tubing may be placed into a tube channel of an infusion pump. The IV tubing may be pressed up against a force sensor using a cover of the infusion pump. This compressive force from the cover may be similar in magnitude to a pressure caused by an occlusion, which may cause the force sensor to transmit force measurements that are incorrectly interpreted as an occlusion being present. The force applied by the cover must therefore be removed from the force sensor output to properly assess the fluid pressure within the IV tubing. However, external factors, such as a height of the intravenous connection to a patient and characteristics of various devices associated with the infusion pump (e.g., a fluid container, a pumping chamber, the IV tubing, etc.) can also influence measurements by the force sensor, thereby affecting the reliability of using the force sensor to determine an occlusion status. In addition, the IV tubing that was originally pressed up against the force sensor may gradually relax over time within the confined tube channel of the infusion pump. This can reduce the pressure measured by the force sensor, which further affects the reliability of this approach for occlusion detection.

One way to overcome or minimize the influence of these external factors is by calculating a force baseline. Such a force baseline may be captured prior to or during the start of an infusion. The force baseline can be subtracted from the measured force to provide a more accurate reading of the forces within the tube itself. The problem with this approach is that these external factors can change over time, so the force baseline may also need to be adjusted to account for the changing external factors. However, adjustments to the force baseline may not be in synchronization with the changes in the external factors. Further, the tools used to make these adjustments may often have difficulty distinguishing changes in the external factors from changes in pressure of the IV tubing. This lag in adjusting the force baseline often leads to false occlusion detection.

Accordingly, a more accurate and reliable method and system for detecting occlusions in infusion pumps is desired.

### SUMMARY

The present disclosure provides a new and innovative method and system for occlusion detection using artificial intelligence. In various embodiments, the device in which occlusion can be detected is an infusion pump. The infusion pump in various embodiments is a peristaltic pump, a syringe pump, or an ambulatory pump configured to deliver a medication to a patient. It should be appreciated that the device is, in various embodiments, any type of medical device, or any other suitable device where detecting any occlusion occurring within the device is crucial for its safe and effective operation.

The disclosed method includes steps to precondition input data from infusion pump pressure samples, steps to train a machine learning model (e.g., a support vector machine) using preconditioned data from reference infusion pump pressure samples, and applying the trained machine learning model to preconditioned input data from an infusion session to detect occlusions in real-time. The preconditioning steps involve receiving one or more types of input data, including, but not limited to (1) a current measurement of a physical force (e.g., a force and/or a pressure detected by a force sensor) within a periodic infusion sample, (2) an initial measurement of the physical force at the start of an infusion (e.g., a reference or baseline force), (3) a metric to represent a long term history of the physical force (e.g., a slope value represented by the current measurement of the physical force over a measurement of the physical force at a distant past), and (4) a metric to represent a short term history of the physical force (e.g., a slope value represented by the current measurement of the physical force over a measurement of the physical force at a near past). The use of different types of input data enable the disclosed machine learning model to differentiate between slowly changing forces, such as tube settling and faster changing forces such as occlusions. Furthermore, the preconditioning steps enable the machine learning model to ideally find statistically significant differences that denote an occlusion scenario over a non-occlusion scenario.

The training of the machine learning model may include receiving a training data set comprising the above described input data (e.g., (1) a current measurement of a physical force, (2) an initial measurement of the physical force at the start of an infusion, (3) a metric to represent a long term history of the physical force, and (4) a metric to represent the short term history of the physical force) for reference infusion events with a known output (e.g., an occlusion or an absence of an occlusion). The received input data set may be arranged as input vectors and associated with their corresponding outputs. For example, a training data set of X, may comprise N input vectors, x₁, ..., x_{N}, with corresponding target values y₁, ..., y_{N}, where yₙ ∈ {-1, 1}. If a given data sample represented by a given input vector, xₙ, did not involve an occlusion, the given input vector may be associated with an output, y = -1. Conversely, if a given data sample represented by a given input vector, xₙ, involved an occlusion, the given input vector may be associated with an output, y = 1.

Given the nature of the occlusion detection in the infusion system, a Support Vector Machine ("SVM") may be used to train the machine learning model. Thus, data sets may be classified into the two groups (e.g., occlusion and no occlusion) and a decision boundary with an optimal boundary may be determined (e.g., that optimizes the margin between occluded and un-occluded data). The parameters for the optimal decision boundary for the training data set may be stored for use during infusion treatments.

The trained machine learning model may be subsequently utilized with a new sample dataset in order to detect when an occlusion occurs. For example, as an infusion pump is configured for a treatment, and force sensors begin measuring physical forces, new values for the above described types of input data are obtained (e.g., (1) a current measurement of a physical force, (2) an initial measurement of the physical force at the start of an infusion, (3) a metric to represent a long term history of the physical force, and (4) a metric to represent the short term history of the physical force). Input vectors may be formed using the input data, and then inputted into the trained machine learning algorithm to determine whether an occlusion has occurred.

In light of the disclosure herein and without limiting the disclosure in any way, in a first aspect of the present disclosure, which may be combined with any other aspect listed herein unless specified otherwise, an infusion pump apparatus for detecting an occlusion includes intravenous ("IV") tubing having a first end that is fluidly coupled to a patient and a second end that is fluidly coupled to a container, a pumping mechanism operable with a portion of the IV tubing for providing controlled delivery of a fluid from the container to the patient, a force sensor coupled to the IV tubing to measure a physical force caused by the fluid, one or more processors, and a memory storing instructions. When executed by the one or more processors, the instructions cause the one or more processors to receive, via the force sensor and during a start of an infusion session, an initial measurement of a physical force caused by fluid motion through the IV tubing, and identify, based on one or more parameters associated with the infusion session, a trained support vector machine for occlusion detection. The one or more processors are also configured to generate, during the infusion session at predetermined intervals, a feature vector comprising the initial measurement of the physical force measured by the force sensor, a current measurement of the physical force measured by the force sensor, a long term change in the measurement of the physical force, and a short term change in the measurement of the physical force. The one or more processors are further configured to input, during the infusion session at the predetermined intervals, the feature vector into the trained support vector machine to output a detection of a presence or an absence of an occlusion in the IV tubing and when the presence of the occlusion is detected, pause or terminate the infusion session.

In accordance with a second aspect of the present disclosure, which may be used in combination with any other aspect listed herein unless stated otherwise, generating the feature vector further comprises receiving, via the force sensor, a current measurement of the physical force, determining, based on a first prior measurement of the physical force, a long term change in the measurement of the physical force, and determining, based on a second prior measurement of the physical force, a short term change in the measurement of the physical force.

In accordance with a third aspect of the present disclosure, which may be used in combination with any other aspect listed herein unless stated otherwise, the instructions, when executed, further cause the one or more processors to train the support vector machine using a training dataset from a plurality of reference infusion sessions sharing the one or more parameters of the infusion session.

In accordance with a fourth aspect of the present disclosure, which may be used in combination with any other aspect listed herein unless stated otherwise, training the support vector machine further comprises receiving, for each of the plurality of reference infusion sessions one or more reference feature vectors corresponding to one or more times during the respective infusion session, wherein a given reference feature vector comprises a reference initial measurement of the physical force at the start of the respective reference infusion session, a reference current measurement of the physical force at a given time of the one or more times during the reference infusion session, a reference long term change in the measurement of the physical force, and a reference short term change in the measurement of the physical force. An indication of a presence or an absence of an occlusion at the one or more times during the respective infusion session is also received. Training the support vector machine also comprises associating, for each of the plurality of reference infusion sessions, the one or more feature vectors with either the indication of the presence of the occlusion or the indication of the absence of the occlusion, determining, based on an optimized margin, a decision boundary for the training dataset, wherein the decision boundary divides the reference feature vectors associated with the indication of the presence of the occlusion from the reference feature vectors associated with the indication of the absence of the occlusion, and generating, based on the decision boundary, the trained support vector machine.

In accordance with a fifth aspect of the present disclosure, which may be used in combination with any other aspect listed herein unless stated otherwise, the long term change in the measurement of the physical force includes a slope value represented by the current measurement of the physical force over a measurement of the physical force at a distant past.

In accordance with a sixth aspect of the present disclosure, which may be used in combination with any other aspect listed herein unless stated otherwise, the distant past includes one or more measurements of the physical force recorded at one to two minutes previously.

In accordance with a seventh aspect of the present disclosure, which may be used in combination with any other aspect listed herein unless stated otherwise, the long term change in the measurement of the physical force includes a slope value that is equal to the current measurement of the physical force minus a measurement of the physical force at a distant past, divided by a number of measurements recorded between the current measurement of the physical force and the measurement of the physical force at the distant past.

In accordance with an eighth aspect of the present disclosure, which may be used in combination with any other aspect listed herein unless stated otherwise, the distant past includes one or more measurements of the physical force recorded at one to two minutes previously.

In accordance with a ninth aspect of the present disclosure, which may be used in combination with any other aspect listed herein unless stated otherwise, the short term change in the measurement of the physical force includes a slope value represented by the current measurement of the physical force over a measurement of the physical force at a near past.

In accordance with a tenth aspect of the present disclosure, which may be used in combination with any other aspect listed herein unless stated otherwise, the near past includes one or more measurements of the physical force recorded at 50 milliseconds to 100 milliseconds previously.

In accordance with an eleventh aspect of the present disclosure, which may be used in combination with any other aspect listed herein unless stated otherwise, the short term change in the measurement of the physical force includes a slope value that is equal to the current measurement of the physical force minus a measurement of the physical force at a near past, divided by a number of measurements recorded between the current measurement of the physical force and the measurement of the physical force at the near past.

In accordance with a twelfth aspect of the present disclosure, which may be used in combination with any other aspect listed herein unless stated otherwise, the near past includes one or more measurements of the physical force recorded at 50 milliseconds to 100 milliseconds previously.

In accordance with a thirteenth aspect of the present disclosure, which may be used in combination with any other aspect listed herein unless stated otherwise, the one or more processors are configured to cause an alert or an alarm to be displayed on a user interface when the presence of the occlusion is detected.

In accordance with a fourteenth aspect of the present disclosure, which may be used in combination with any other aspect listed herein unless stated otherwise, an infusion system for detecting an occlusion includes a force sensor coupled to IV tubing to measure a physical force caused by a fluid within the IV tubing, the IV tubing having a first end that is fluidly coupled to a patient and a second end that is fluidly coupled to a container. The system also includes an infusion device communicatively coupled to the force sensor. The infusion device includes a pumping mechanism operable with a portion of the IV tubing for providing controlled delivery of a fluid from the container to the patient, a user interface, one or more processors, and a memory storing a plurality of trained support vector machines and instructions that, when executed by the one or more processors, cause the one or more processors to receive, via the force sensor and during a start of an infusion session, an initial measurement of a physical force caused by fluid motion through the IV tubing and identify, based on one or more parameters associated with the infusion session, a trained support vector machine for occlusion detection among the plurality of the trained support vector machines. The one or more processors are also configured to generate, during the infusion session at predetermined intervals, a feature vector comprising the initial measurement of the physical force measured by the force sensor, a current measurement of the physical force measured by the force sensor, a long term change in the measurement of the physical force, and a short term change in the measurement of the physical force. The one or more processors are further configured to input, during the infusion session at the predetermined intervals, the feature vector into the trained support vector machine to output a detection of a presence or an absence of an occlusion in the IV tubing and when the presence of the occlusion is detected, cause an alert or an alarm to be displayed on the user interface that is indicative of the occlusion.

In accordance with a fifteenth aspect of the present disclosure, which may be used in combination with any other aspect listed herein unless stated otherwise, the one or more processors are configured to pause or terminate the infusion session when the presence of the occlusion is detected.

In accordance with a sixteenth aspect of the present disclosure, which may be used in combination with any other aspect listed herein unless stated otherwise, the force sensor is integrally formed or fluidly connected to the IV tubing.

In accordance with a seventeenth aspect of the present disclosure, which may be used in combination with any other aspect listed herein unless stated otherwise, generating the feature vector further comprises receiving, via the force sensor, a current measurement of the physical force, determining, based on a first prior measurement of the physical force, a long term change in the measurement of the physical force, and determining, based on a second prior measurement of the physical force, a short term change in the measurement of the physical force.

In accordance with an eighteenth aspect of the present disclosure, which may be used in combination with any other aspect listed herein unless stated otherwise, the instructions, when executed, further cause the one or more processors to train the support vector machine using a training dataset from a plurality of reference infusion sessions sharing the one or more parameters of the infusion session.

In accordance with a nineteenth aspect of the present disclosure, which may be used in combination with any other aspect listed herein unless stated otherwise, training the support vector machine further comprises receiving, for each of the plurality of reference infusion sessions one or more reference feature vectors corresponding to one or more times during the respective infusion session, wherein a given reference feature vector comprises a reference initial measurement of the physical force at the start of the respective reference infusion session, a reference current measurement of the physical force at a given time of the one or more times during the reference infusion session, a reference long term change in the measurement of the physical force, and a reference short term change in the measurement of the physical force. An indication of a presence or an absence of an occlusion at the one or more times during the respective infusion session is also received. Training the support vector machine also comprises associating, for each of the plurality of reference infusion sessions, the one or more feature vectors with either the indication of the presence of the occlusion or the indication of the absence of the occlusion, determining, based on an optimized margin, a decision boundary for the training dataset, wherein the decision boundary divides the reference feature vectors associated with the indication of the presence of the occlusion from the reference feature vectors associated with the indication of the absence of the occlusion, and generating, based on the decision boundary, the trained support vector machine.

In accordance with a twentieth aspect of the present disclosure, which may be used in combination with any other aspect listed herein unless stated otherwise, an infusion pump method for detecting an occlusion includes receiving, in a processor of an infusion device from a force sensor and during a start of an infusion session, an initial measurement of a physical force caused by fluid motion through IV tubing having a first end that is fluidly coupled to a patient and a second end that is fluidly coupled to a container and identifying, via the processor based on one or more parameters associated with the infusion session, a trained support vector machine for occlusion detection. The method also includes generating, via the processor during the infusion session at predetermined intervals, a feature vector comprising the initial measurement of the physical force measured by the force sensor, a current measurement of the physical force measured by the force sensor, a long term change in the measurement of the physical force, and a short term change in the measurement of the physical force. The method further includes inputting, via the processor during the infusion session at the predetermined intervals, the feature vector into the trained support vector machine to output a detection of a presence or an absence of an occlusion in the IV tubing and when the presence of the occlusion is detected, pausing or terminating the infusion session via the processor.

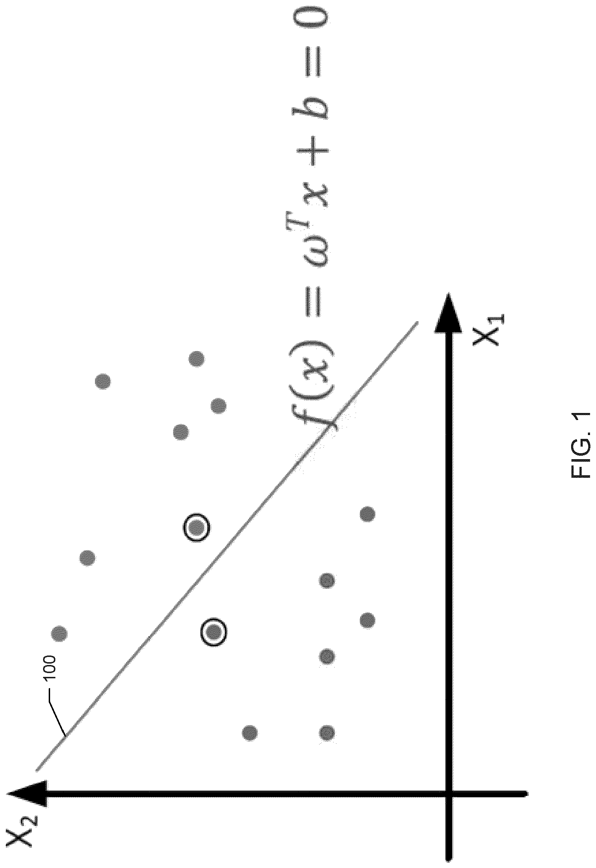

In accordance with a twenty-first aspect of the present disclosure, any of the structure, functionality, and alternatives disclosed in connection with any one or more of Figs. 1 to 9 may be combined with any other structure, functionality, and alternatives disclosed in connection with any other one or more of Figs. 1 to 9.

In light of the present disclosure and the above aspects, it is therefore an advantage of the present disclosure to provide an infusion system configured to detect occlusions while compensating for long and short term variations within IV tubing or the infusion system.

It is another advantage of the present disclosure to train and use support vector machines for detecting occlusions in IV tubing.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

### BRIEF DESCRIPTION OF THE DRAWINGS

FIG. 1 is a graph illustrating an example two-class classification for occlusion detection, according to an embodiment of the present disclosure.
FIG. 2 is a graph illustrating a maximum margin classifier, according to an embodiment of the present disclosure.
FIG. 3 is a matrix showing an example occlusion training dataset, according to an embodiment of the present disclosure.
FIG. 4 is a vector showing an example target value for the example occlusion training dataset, according to an example embodiment of the present disclosure.
FIG. 5 is a flow chart illustrating a method for training a model for occlusion detection, according to an embodiment of the present disclosure.
FIG. 6 is a flow chart illustrating a method for occlusion detection in infusion systems using a trained model, according to an embodiment of the present disclosure.
Figs. 7 and 8 are diagrams of an infusion device that is configured to use a trained support vector machine to perform the method of Fig. 6, according to an example embodiment of the present disclosure.
Fig. 9 is a diagram of an infusion system configured to perform the operations described in connection with Fig. 5, according to an example embodiment of the present disclosure.

### DETAILED DESCRIPTION

In-line occlusion detection is critical for the safe and effective operation of infusion systems. The most common method for occlusion detection utilizes a force sensor to detect in-line pressure (e.g., a pressure caused by fluid within a IV tubing coupled to an infusion device). The detected in-line pressure can indirectly indicate an occlusion status (e.g., a presence or an absence of an occlusion), based on whether or not the detected in-line pressure reaches or exceeds a preset threshold. However, false alarms of occlusion can occur for a variety of factors, including, but not limited to, variations in infusion systems and variations regarding the patient. The present disclosure provides more robust, accurate, and/or reliable systems and methods for occlusion detection in infusion systems. Furthermore, the present disclosure includes novel and nonobvious systems and methods for preconditioning data, and using particular artificial intelligence models (e.g., support vector machines) specifically suited to detect upstream and downstream occlusions which take place in the infusion systems.

Some embodiments for occlusion detection in infusion systems may include various parts, namely, preconditioning input data, training a machine learning model for occlusion detection, and applying the machine learning model. In the following paragraphs, these parts will be discussed with more detail.

A method for preconditioning input data may begin by receiving data from a force sensor. As previously discussed, a force sensor may be coupled to IV tubing of an infusion device to measure a physical force caused by fluid within the tubing. The fluid is delivered by an infusion device for infusion into a patient. In some embodiments, measurements of physical forces from the force sensor may be periodically sampled (e.g., measurements may be obtained in predetermined intervals of time). These sampled measurement readings may be fed into the occlusion detection models that are further described herein. Furthermore, these samples may be buffered, averaged, accumulated, and/or counted (e.g., preconditioned) to determine a baseline measurement of the physical force and a threshold value for the physical force. For example, data received from the force sensors may be preconditioned to represent:
(A) a current measurement of a physical force at periodic intervals;
(B) an initial reference at the start of the infusion - the intent is to provide the algorithm with a pump-to-pump, setup-to-setup, reference for an infusion treatment;
(C) an indicator of a long-term change in the physical force (e.g., to provide the machine learning model an indicia of the physical force over a long enough period to make significant rises or falls in the physical force obvious); and
(D) an indicator of a short-term change in the physical force (e.g., to provide the machine learning model an indicia of the current progress of the physical force (e.g., how fast the physical force is rising or falling).

The input for "(A)" may be a measurement of the physical force as part of the periodic sampling in real time.

The input for "(B)" may be a measurement of the physical force at the start of the infusion session.

The inputs for "(C)" and "(D)" may comprise the slopes in the current measurement of the physical force and the measurement of the physical force sometime in the past. Slopes may be used, for example, to provide for easily calculated values, which minimize the amount of information that needs to be stored in the infusion system and/or infusion device (e.g., at the microcontroller, memory, and/or electronic storage device).

For the input for "(C)", a measurement of a physical force 1 - 2 minutes in the past may be held and used to calculate a slope with the current measurement of the physical force. In other embodiments, the input for (C) may use a measurement of a physical force that is between thirty seconds to ten minutes in the past. In an example, the input (C) may be computed as: ${Slope}_{1 MIN} = \left({X}_{CURRENT}-{X}_{1 MINREF}\right) / 12000 ,$ where X_{CURRENT} is the current measurement of the physical force and X_{1MINREF} is the measurement of the physical force from 1 to 2 minutes in the past. The difference may be divided by a value (e.g., 12000) to represent the number of samples taken in 1 minute at a 5 millisecond ("ms") sample rate of the force sensors (e.g., an interval rate).

For "(D)", a sample measurement of physical force 50 - 100ms in the past may be held and used to calculate a slope with the current sample measurement of the physical force. Thus, the input (D) may be computed as: ${Slope}_{50 MS} = \left({X}_{CURRENT}-{X}_{50 MSREF}\right) / 10 ,$ where X_{CURRENT} is the current ADC sample and X_{50MSREF} is the reference ADC sample from 50 to 100 milliseconds in the past. The 10 value may represent the number of samples taken in 50ms at the 5ms sample rate of the force sensors. In other embodiments, the input for (D) may use a measurement of a physical force that is between 1ms to 5 seconds in the past.

The preconditioned inputs representing a long-term and a short-term change in the physical force (e.g., via slope information) provides the machine learning model information which could help it differentiate between slowly changing forces, such as tube settling, and faster changing forces, such as occlusions. Although it is understood that not all occlusion pressure changes occur quickly, providing the machine learning model at least two such above described inputs that capture the changes in the physical force may allow the machine learning model to find statistically significant differences that denote an occlusion scenario over a non-occlusion scenario. Furthermore, the two above described inputs capturing changes in the physical force can also be used to detect a scenario where IV tubing (e.g., an infusion passageway) may be occluded prior to starting an infusion session or treatment.

FIG. 1 is a graph illustrating an example two-class classification for occlusion detection, according to an embodiment of the present disclosure. A support vector machine ("SVM") is an example of a machine learning tool that can be customized and applied for occlusion detection, given the nature of occlusion detection in infusion systems. As can be seen in FIG. 1, a decision boundary 100 is chosen to be the one for which the margin is maximized. The linear model form for the decision boundary can be expressed as the following equation: $f \left(x\right) = {ω}^{T} x + b = 0 ,$ where X is the training occlusion data set comprising N input vectors x₁, ..., x_{N}, with corresponding target values y₁, ..., y_{N}, where yₙ ∈ {-1, 1}. Occlusion and non-occlusion are represented in yₙ by 1 and -1, respectively. The parameters *ω* and b are chosen such that formula 1 satisfies f(xₙ) > 0 for points having yₙ = 1 and f(xₙ) < 0 for points having yₙ =-1. The support vectors are the vectors having closest distance to the decision boundary 100 in FIG. 1.

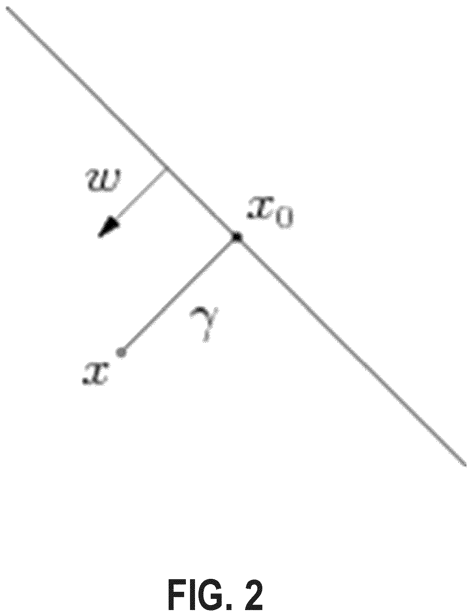

FIG. 2 is a graph illustrating a maximum margin classifier. As shown in FIG. 2, to optimize the margin between occluded and un-occluded data and further derive *ω*, the distance between the support vector x and the decision boundary is maximized. The optimization problem may involve a minimization of ∥*ω*∥, which can be accomplished via the following equation: $γ = \frac{{ω}^{T} x + b}{\left‖ω\right‖} ,$ where Y is the distance between the support vector x and the decision boundary.

A transformed optimization problem may be represented as the following equation: $arg {min}_{ω , b} \frac{1}{2} {\left‖ω\right‖}^{2} s . t . , {y}_{i} \left({ω}^{T}{x}_{i}+b\right) \geq 1 , i = 1 , … , n$

In order to solve this constrained optimization problem, a Lagrange duality method as shown below, may be utilized: $L \left(ω, b, α\right) = \frac{1}{2} {\left‖ω\right‖}^{2} - {\sum }_{i = 1}^{n} {α}_{i} \left({y}_{i}\left({ω}^{T}{x}_{i}+b\right)-1\right) ,$ where αₙ comprise Lagrange multipliers that satisfies αₙ ≥ 0. Further, by setting the partial derivatives of *L*(*ω*, *b, α*) with respect to *ω* and b equal to zero, *ω* and b can be eliminated from *L*(*ω*, *b, α*) to give the dual representation of the maximum margin problem, in which maximized α as can expressed as: $\frac{\partial L}{\partial ω} = 0 \to ω = {\sum }_{i = 1}^{n} {α}_{i} {y}_{i} {x}_{i} and \frac{\partial L}{\partial b} = 0 \to b = {\sum }_{i = 1}^{n} {α}_{i} {y}_{i} = 0 .$

Incorporating the above formula into the formula for the Lagrange duality method may yield: ${max}_{α} {\sum }_{i = 1}^{n} {α}_{i} - \frac{1}{2} {\sum }_{i , j = 1}^{n} {α}_{i} {α}_{j} {y}_{i} {y}_{j} {x}_{i}^{T} {x}_{j}$ $s . t . , {α}_{i} \geq 0 , i = 1 , … , n$ ${\sum }_{i = 1}^{n} {α}_{i} {y}_{i} = 0$

The aforementioned method may be used to solve a linear classification problem. In order to build a robust occlusion algorithm, a non-linear occlusion dataset may be assumed to be present. Then, a kernel function can be introduced herein to map the current occlusion data to higher dimensions in order to determine the decision boundary. The following formula may be used to introduce the kernel function *κ* to replace ${x}_{i}^{T} {x}_{j}$ in the aforementioned method: ${max}_{α} {\sum }_{i = 1}^{n} {α}_{i} - \frac{1}{2} {\sum }_{i , j = 1}^{n} {α}_{i} {α}_{j} {y}_{i} {y}_{j} κ \left({x}_{i}, {x}_{j}\right) ,$ where a kernel function can be polynomial, linear, and Gaussian functions as expressed below:

| | |
|---|---|
| Polynomial: | *κ*(*x*₁,*x*₂) *=* (〈*x*₁,*x*₂〉 + *R*)*^{d}*, *R and d are mapping parameters* |
| Linear: | *κ*(*x*₁,*x*₂) *=* 〈*x*₁,*x*₂〉 |
| Gaussian: | $κ \left({x}_{1}, {x}_{2}\right) = exp \left(-\frac{{\left‖{x}_{1}-{x}_{2}\right‖}^{2}}{2 {σ}^{2}}\right)$ , ***σ*** is the attenuation parameter. |

For ease of explanation, a linear kernel function is utilized in the foregoing sections to demonstrate the various embodiments described herein.

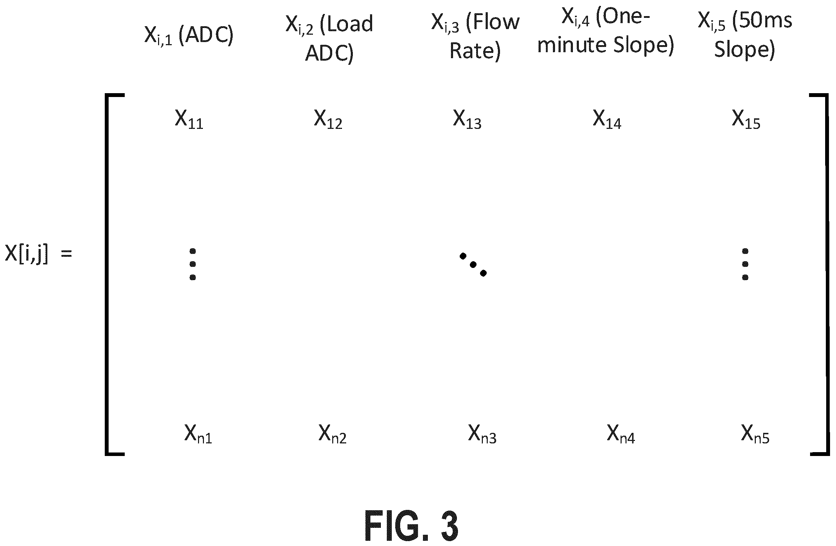

FIG. 3 is a matrix showing an example occlusion detection training dataset, according to an embodiment of the present disclosure. As previously discussed, a support vector machine ("SVM") can be utilized to build a customized model for occlusion detection. The occlusion detection training dataset, x, is a matrix that comprises N input vectors x₁, ..., x_{N}, and can represent occlusion related parameters, such as ADC counts, slopes, flowrates, current drain, temperature, etc. As can be seen in FIG. 3, a five parameters have been chosen for vectors X for demonstration purposes: a current measurement of an ADC count, a load ADC count, a flow rate, a long term change in the measurement of a physical force (e.g., a one minute slope), and a long term change in the measurement of a physical force (e.g., a 50ms slope). In the example occlusion detection training dataset shown in FIG. 3, a five-dimension dataset is chosen for the training. The five-dimensional dataset may also be used for the application of the trained model. As previously discussed, parameters may also or alternatively include, for example, a current measurement of a physical force at periodic intervals, an initial measurement of the physical force (e.g., at the start of an infusion session), an indicator of a long term change in the measurement of the physical force, an indicator of a short term change in the measurement of the physical force, etc. Thus, based on the numbers of parameters being used, the dimensionality of a dataset may correspond accordingly.

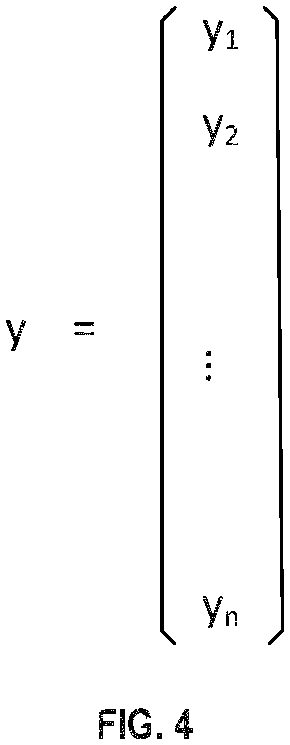

FIG. 4 is a vector showing example target values for the example occlusion training dataset of FIG. 3, according to an example embodiment of the present disclosure. In order to facilitate supervised machine learning, a training dataset may have known target values indicating whether an occlusion was present. Thus, each target value in the vector shown in FIG. 4 corresponds to a five-dimensional feature vector in FIG. 3.

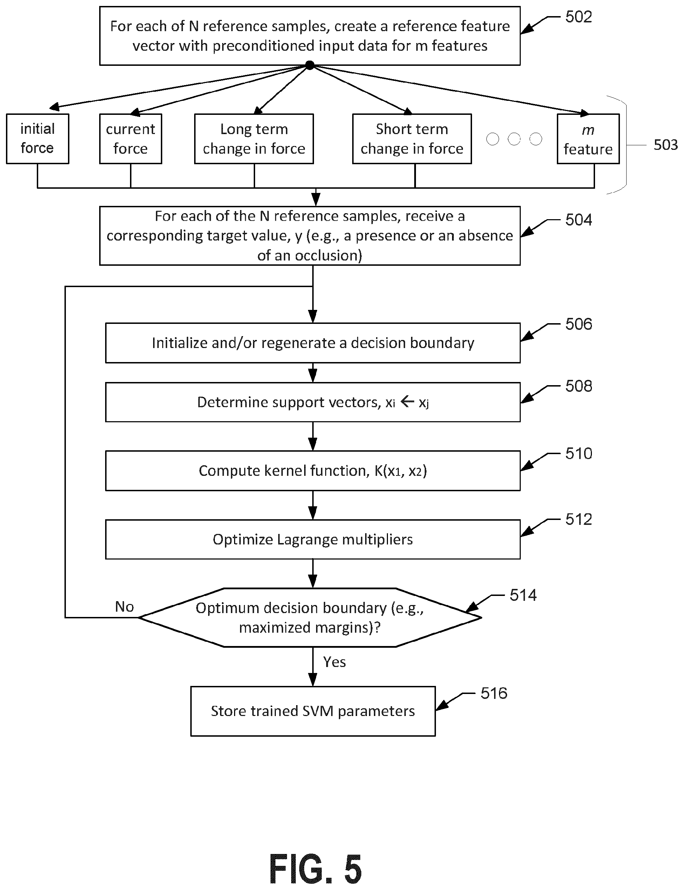

FIG. 5 is a flow chart illustrating a method for training a model for occlusion detection, according to an embodiment of the present disclosure. The method may be performed by one or more processors associated with an infusion system. The method may be performed after preconditioning input data received from the force sensor associated with the input system. The training may involve an occlusion detection training dataset (e.g., as shown in FIGS. 3 and 4) obtained from a plurality of reference samples. The reference sample may be from various patients, past data, external libraries or databases, etc.

At step 502, the infusion system may create, for each of a plurality of reference samples (e.g., N reference samples), a reference feature vector with preconditioned input data for m features. Just as the input feature vector described in FIG. 3 was five-dimensional as a result of there being five parameters, the reference feature vector may be m-dimensional as a result of m features. As an example, the m features may include, but are not limited to, an initial measurement of a physical force at the start of the reference infusion session, a current measurement of a physical force during periodic intervals of the reference infusion session, a long term change in the physical force, a short term change in the physical force, etc., as shown in marker 503.

At step 504, the infusion system may receive, for each of the N reference samples, a corresponding target value, y, indicating either a presence or an absence of an occlusion. Just as the target values in FIG. 4 indicated either a presence or an absence of an occlusion for the training dataset in FIG. 3, the target values, y, in step 504 may indicate whether each sample received in step 502 indicated a presence or an absence of an occlusion. The target value, y, may comprise of N values corresponding to the N reference samples. Given the binary nature of the indication (e.g., a presence or an absence of an occlusion), the target value may comprise, for example, -1 to indicate an absence of an occlusion or 1, to indicate a presence of an occlusion.

At step 506, the infusion system may initialize and/or regenerate a decision boundary (e.g., the decision boundary 100 of Fig. 1). When the training dataset is mapped, the infusion system may use the training method described in FIG. 5 to learn how to divide data points corresponding to those having an indication of an occlusion from those having an indication of no conclusion (e.g., via the decision boundary 100). In the process of learning, the infusion system may initially create a decision boundary that may not necessarily be the most optimal. Based on the decision boundary 100, the infusion system may determine support vectors, xᵢ ← xⱼ for each of the reference samples, N, at step 508.

At step 510, a kernel function, K(x₁, x₂) may be computed. In some embodiments, the infusion system may optimize Lagrange multipliers, e.g., to determining the optimal decision boundary.

At step 514, the infusion system may determine whether the decision boundary generated for the dataset is optimal (e.g., whether the decision boundary has the maximum margins from the binarily classified data points). If it is found that the decision boundary is not the most optimal, steps 506 through 514 may be repeated.

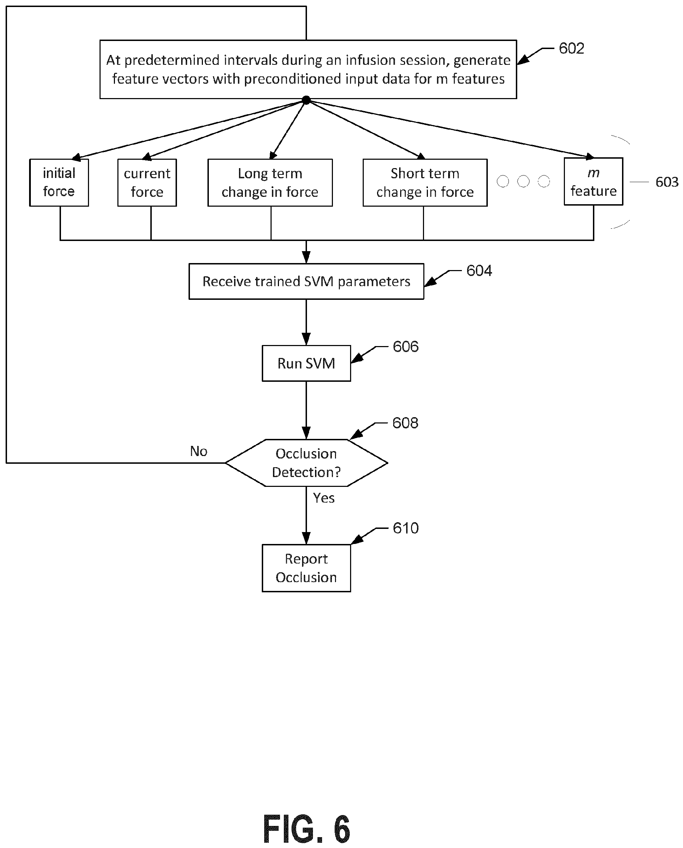

Otherwise, the trained support vector machine, with its learned parameters may be stored in step 516, e.g., in an electronic storage medium associated with the infusion system. For example, the previously discussed decision boundary vectors ω and b can be computed through the training method shown in FIG. 5. The stored parameters for the trained support vector machine, ω and b, can then be utilized during an application of the trained support vector machine during a new infusion session for real-time occlusion detection, as will be shown in FIG. 6.

FIG. 6 is a flow chart illustrating a method for occlusion detection in infusion systems using a trained model, according to an embodiment of the present disclosure.

At step 602, the infusion system may generate, at predetermined intervals during an infusion session, feature vectors with preconditioned input data for m features. The m features may include, for example, an initial measurement of a physical force at the start of the reference infusion session, a current measurement of a physical force during periodic intervals of the reference infusion session, a long term change in the physical force, a short term change in the physical force, etc., as shown in marker 603.

At step 604, the infusion system may receive the trained support vector machine (e.g., the trained parameters, ω and b). In some embodiments, there may be a plurality of trained support vector machines stored externally, with each trained support vector machine corresponding to specific parameters that are unique to an infusion system. An appropriate trained support vector machine may be identified and its trained parameters retrieved, using the parameters unique to the infusion system. At step 606, the infusion system may run the support vector machine with its trained parameters, ω and b, using the feature vector formed during the specific time interval from step 602.

The support vector machine can thus detect in real-time, at step 608, whether there is an occlusion. If there is no indication of an occlusion, steps 602 through 608 may be repeated, e.g., based on preconditioned input data at the next time interval of the infusion session. Otherwise, at step 610, the infusion system may report the occlusion. For example, the infusion system may display an indication of the occlusion on a display device. Also or alternatively, the infusion system may terminate the infusion session upon detecting the occlusion.

### Example Infusion System and Infusion Device

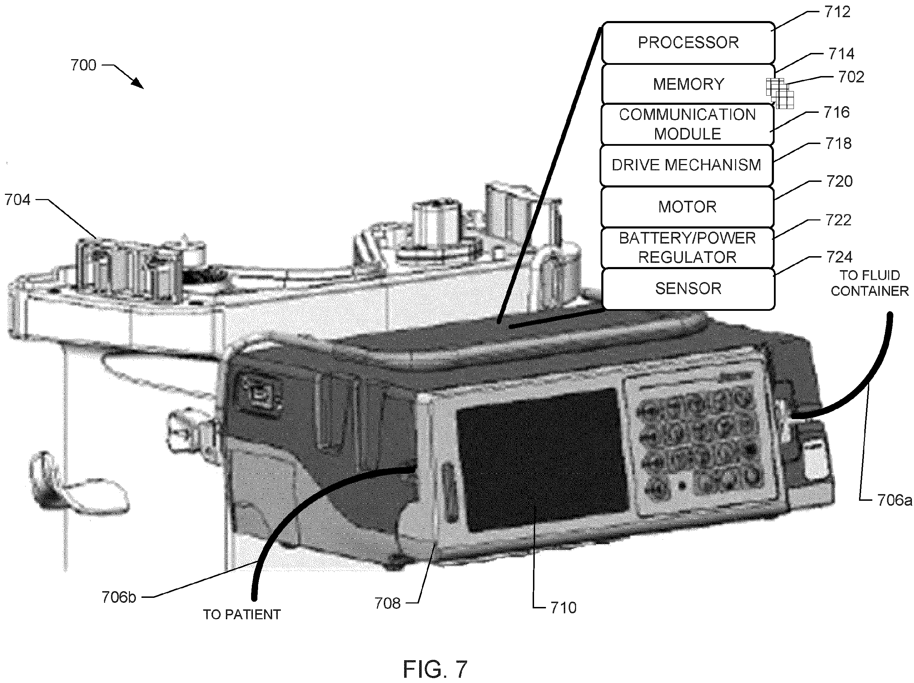

Fig. 7 is a diagram of an infusion device 700 that is configured to use a trained support vector machine 702 to perform the method of Fig. 6, according to an example embodiment of the present disclosure. The infusion device 700 is an infusion pump, such as a syringe pump, an ambulatory pump, or a peristaltic pump. The infusion device 700 is connected to a rack 704 for support.

The infusion device 700 is configured to receive IV tubing 706. In an example, a cover 708 of the infusion device 700 opens, enabling the IV tubing 706 to be inserted. A first end of the IV tubing 706a is fluidly coupled to a fluid container that holds a drug, medication, or other fluid for an infusion treatment. A second end of the IV tubing 706b is fluidly coupled to a patient via an intravenous connection.

The infusion device 700 includes a user interface 710 for receiving operator inputs (e.g., a flow rate) such as the one or more parameters discussed above. The user interface 710 also displays information including a status of an infusion treatment and alarms/alerts indicative of an occlusion. The user interface 710 includes a touchscreen and a keypad. In other embodiments, the user interface 710 may include only a touchscreen or a keypad.

The infusion device 700 of Fig. 7 also includes a processor 712, a memory 714, and a communication module 716. While one processor 712 is shown, the infusion device 700 may include a plurality of processors. The processor 712 includes a controller, a logic device, etc. configured to execute the trained support vector machine 702 (e.g., an algorithm) stored in the memory 714. The processor 712 is also configured to execute one or more instructions stored in the memory 714 that, when executed by the processor 712, cause the processor 712 to perform the operations described herein to provide an infusion treatment. The memory 714 includes any memory device including read only memory, flash memory, random access memory, a hard disk drive, a solid state drive, etc.

The communication module 716 is configured for wireless and/or wired communication with a network, such as the Internet, a cellular network, and/or a local hospital network. The communication module 716 may be configured, for example, for Wi-Fi or Ethernet communication. In the illustrated example, the communication module 716 is configured to receive the trained support vector machine 702 (including trained parameters, ω and b) from a server or clinician computer via a network. In other examples, the processor 712 may perform the method of Fig. 5 to train the support vector machine 702. The communication module 716 may also receive the one or more parameters specifying an infusion treatment to be performed. Further, the communication module 716 may transmit alert or alarm messages to a server when an occlusion is detected.

The infusion device 700 of Fig. 7 further includes a drive mechanism 718, a motor 720, a battery/power regulator 722, and a sensor 724. Together, the drive mechanism 718 and the motor 720 comprise a pumping mechanism. The processor 712 is configured to transmit signals or commands to the motor 720, which cause the motor 720 to rotate or otherwise operate in a certain direction and speed. The movement or rotation of a drive shaft of the motor 720 causes the drive mechanism 718 to actuate or otherwise provide force on the IV tubing 706 (or a fluid container in alternative embodiments where a fluid container is placed inside the infusion device). The drive mechanism 718 may include finger actuators or a rotary actuator that apply pressure on the IV tubing 706 to deliver fluid from the fluid container to a patient for an infusion treatment. The drive mechanism 718 and the motor 720 are collectively configured to provide precise control of fluid delivery between 0.1 milliliters/hour up to 1000 milliliters/hour.

For a syringe pump, the drive mechanism 718 may include a piston or other actuator that pushes on a plunger of a syringe. In some embodiments, the motor 720 may rotate a drive screw, which causes the drive mechanism 718 to apply force on the plunger.

The battery/power regulator 722 is configured to provide electrical power for the infusion device 700. A power regulator converts outlet based AC power into DC power. A battery provides constant DC power.

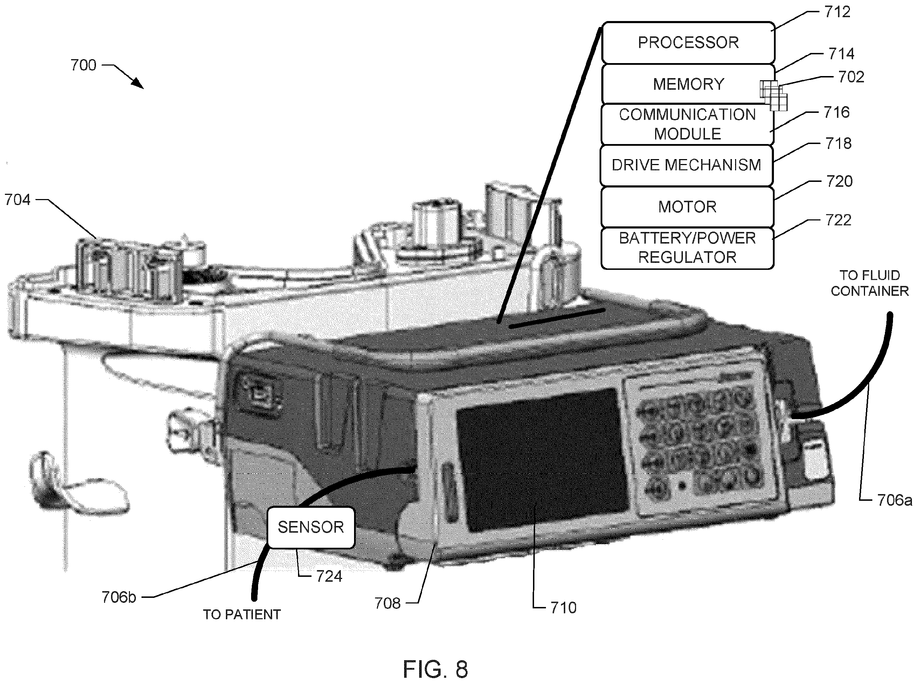

The force sensor 724 is configured to measure a pressure exerted by the IV tubing 706. The force sensor 724 may include a piezo-resistive sensor, a capacitance-based force sensor, a membrane sensor, a strain gauge, etc. In the illustrated example, the force sensor 724 is located within the infusion device 700. Fig. 8 shows an alternative embodiment where the force sensor 724 is located external to the infusion device 700. In these alternative embodiments, the force sensor 724 may be connected via a wire (e.g., a universal serial bus or a serial connection) to the infusion device 700. Alternatively, the force sensor 724 may be connected via a Bluetooth^{®}, Zigbee^{®}, or other wireless connection to the infusion device 700. In some embodiments, the force sensor 724 may include a pressure pod that is integrated with the IV tubing.

In some embodiments, the processor 712 of Figs. 7 and 8 is configured to perform the method described in connection with Fig. 6. In these embodiments, the processor 712 receives measurements from the force sensor 724 of a physical force imparted by the IV tubing 706 that is caused by pumped fluid from the fluid container. The processor 712 selects the trained support vector machine 702 based on the programmed infusion treatment. The processor 712 then records sampled force or pressure measurements from the force sensor 724 and calculates slopes for long-term and short-term changes in the physical force within the IV tubing 706 (e.g., (A), (B), (C), and (D) discussed above). The processor 712 uses (A), (B), (C), and (D) to create a feature vector that is input into the selected trained support vector machine 702. The processor 712 then receives an output from the trained support vector machine 702 to indicate whether an occlusion is detected. When an occlusion is detected, the processor 712 may cause an alert and/or an alarm to be displayed on the user interface 710. Further, the processor 712 may also cause a message indicative of the occlusion to be transmitted to a network via the communication module 716. The processor 712 may perform the method described in connection with Fig. 6 for every new force measurement. Alternatively, the processor 712 may perform the method at programmed periodic intervals, such as every 50 milliseconds, 100 milliseconds, 500 milliseconds, 1 second, 2 seconds, etc.

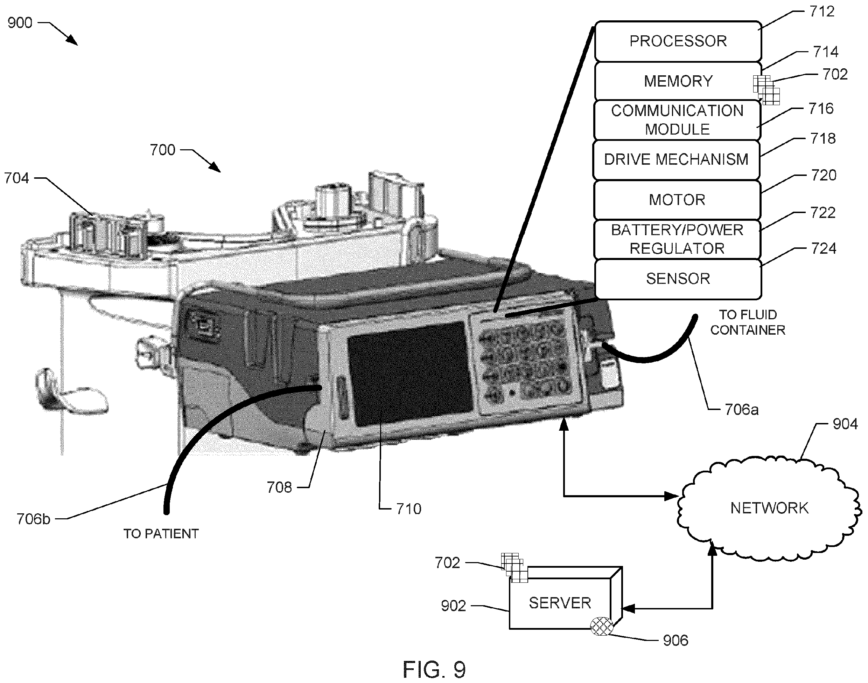

Fig. 9 is a diagram of an infusion system 900 configured to perform the operations described in connection with Fig. 5, according to an example embodiment of the present disclosure. The infusion system 900 includes the infusion device 700 of Figs. 7 and 8. The infusion system 900 also includes a server 902 that is connected to the infusion device 700 via a network 904, which may include any cellular, wide area, and/or local area network. The server 902 may be part of a heath information system and include a clinician computer.

In the illustrated example, the server 902 receives training data 906, such as the N reference samples discussed above. The training data 906 may be input into the server 902 from manually obtained data. Additionally or alternatively, the training data 906 may be received from one or more infusion devices including the infusion device 700.

As discussed above, the server 902 is configured to create one or more trained support vector machines 702 for specific parameters of infusion treatments using the training data 906. The server 902 may transmit the trained support vector machines 702 to the infusion device 700 via the network 904. Alternatively, the server 902 may receive one or more parameters (e.g., a flow rate) from the infusion device 700 before a treatment is to be begin. The server 902 selects the trained support vector machine 702 that matches or corresponds to the received infusion parameters and transmits the selected trained support vector machine 702 to the infusion device 700 for occlusion detection.

### Conclusion

It should be understood that various changes and modifications to the presently preferred embodiments described herein will be apparent to those skilled in the art. Such changes and modifications can be made without departing from the scope of the present subject matter and without diminishing its intended advantages. It is therefore intended that such changes and modifications be covered by the appended claims.

## Claims

1. An infusion system for detecting an occlusion, the system comprising:
a force sensor (724) coupled to IV tubing to measure a physical force caused by a fluid within the IV tubing, the IV tubing having a first end that is fluidly coupled to a patient and a second end that is fluidly coupled to a container; and
an infusion device (700) communicatively coupled to the force sensor, the infusion device including:
a pumping mechanism (718) operable with a portion of the IV tubing for providing controlled delivery of a fluid from the container to the patient,
a user interface (710),
one or more processors (712), and
a memory (714) storing a plurality of trained support vector machines (702) and instructions that, when executed by the one or more processors, cause the one or more processors to:
receive, via the force sensor and during a start of an infusion session, an initial measurement of a physical force caused by fluid motion through the IV tubing;
select, based on one or more parameters associated with the infusion session, a trained support vector machine for occlusion detection among the plurality of the trained support vector machines;
generate, during the infusion session at predetermined intervals, a feature vector configured as an input for the selected trained support vector machine, the feature vector comprising:
the initial measurement of the physical force measured by the force sensor,
a current measurement of the physical force measured by the force sensor,
a long term change in the measurement of the physical force, and
a short term change in the measurement of the physical force;
input, during the infusion session at the predetermined intervals, the feature vector into the trained support vector machine to output a detection of a presence or an absence of an occlusion in the IV tubing; and
when the presence of the occlusion is detected, cause an alert or an alarm to be displayed on the user interface that is indicative of the occlusion.

2. The system of Claim 1, wherein the one or more processors are configured to pause or terminate the infusion session when the presence of the occlusion is detected.

3. The system of Claims 1 or 2, wherein the force sensor is integrally formed or fluidly connected to the IV tubing.

4. The system of Claims 1, 2, or 3 wherein generating the feature vector further comprises:
receiving, via the force sensor, the current measurement of the physical force;
determining, based on a first prior measurement of the physical force, the long term change in the measurement of the physical force; and
determining, based on a second prior measurement of the physical force, the short term change in the measurement of the physical force.

5. The system of Claims 1, 2, 3, or 4 wherein the instructions, when executed, further cause the one or more processors to train the support vector machine using a training dataset from a plurality of reference infusion sessions sharing the one or more parameters of the infusion session.

6. The system of Claim 5, wherein training the support vector machine further comprises:
receiving, for each of the plurality of reference infusion sessions:
one or more reference feature vectors corresponding to one or more times during the respective infusion session, wherein a given reference feature vector comprises:
a reference initial measurement of the physical force at the start of the respective reference infusion session,
a reference current measurement of the physical force at a given time of the one or more times during the reference infusion session,
a reference long term change in the measurement of the physical force, and
a reference short term change in the measurement of the physical force, and
an indication of a presence or an absence of an occlusion at the one or more times during the respective infusion session;
associating, for each of the plurality of reference infusion sessions, the one or more feature vectors with either the indication of the presence of the occlusion or the indication of the absence of the occlusion;
determining, based on an optimized margin, a decision boundary for the training dataset, wherein the decision boundary divides the reference feature vectors associated with the indication of the presence of the occlusion from the reference feature vectors associated with the indication of the absence of the occlusion; and
generating, based on the decision boundary, the trained support vector machine.

7. The system of Claims 1, 2, or 6, wherein the long term change in the measurement of the physical force includes a slope value represented by the current measurement of the physical force over a measurement of the physical force at a distant past.

8. The system of Claim 7, wherein the distant past includes one or more measurements of the physical force recorded at one to two minutes previously.

9. The system of Claims 1, 2, or 6, wherein the long term change in the measurement of the physical force includes a slope value that is equal to the current measurement of the physical force minus a measurement of the physical force at a distant past, divided by a number of measurements recorded between the current measurement of the physical force and the measurement of the physical force at the distant past.

10. The system of Claim 9, wherein the distant past includes one or more measurements of the physical force recorded at one to two minutes previously.

11. The system of Claims 1, 2, 4, or 6, wherein the short term change in the measurement of the physical force includes a slope value represented by the current measurement of the physical force over a measurement of the physical force at a near past.

12. The system of Claim 11, wherein the near past includes one or more measurements of the physical force recorded at 50 milliseconds to 100 milliseconds previously.

13. The system of Claims 1, 2, 4, or 6, wherein the short term change in the measurement of the physical force includes a slope value that is equal to the current measurement of the physical force minus a measurement of the physical force at a near past, divided by a number of measurements recorded between the current measurement of the physical force and the measurement of the physical force at the near past.

14. The system of Claim 13, wherein the near past includes one or more measurements of the physical force recorded at 50 milliseconds to 100 milliseconds previously.

15. An infusion pump method for detecting an occlusion, the method comprising:
receiving, in a processor (712) of an infusion device (700) from a force sensor (724) and during a start of an infusion session, an initial measurement of a physical force caused by fluid motion through IV tubing having a first end that is fluidly coupled to a patient and a second end that is fluidly coupled to a container;
generating, via the processor during the infusion session at predetermined intervals, a feature vector configured as an input for a trained support vector machine (702), the feature vector comprising:
the initial measurement of the physical force measured by the force sensor,
a current measurement of the physical force measured by the force sensor,
a long term change in the measurement of the physical force, and
a short term change in the measurement of the physical force;
inputting, via the processor during the infusion session at the predetermined intervals, the feature vector into the trained support vector machine to output a detection of a presence or an absence of an occlusion in the IV tubing; and
when the presence of the occlusion is detected, pausing or terminating the infusion session via the processor.

## Patentansprüche

1. Infusionssystem zum Erkennen einer Okklusion, wobei das System umfasst:
einen Kraftsensor (724), der mit einem IV-Schlauch gekoppelt ist, um eine physikalische Kraft zu messen, die durch ein Fluid innerhalb des IV-Schlauchs verursacht wird, wobei der IV-Schlauch ein erstes Ende, das fluidisch mit einem Patienten gekoppelt ist, und ein zweites Ende aufweist, das fluidisch mit einem Behälter gekoppelt ist; und
eine Infusionsvorrichtung (700), die zu Kommunikationszwecken mit dem Kraftsensor gekoppelt ist, wobei die Infusionsvorrichtung einschließt:
ein Pumpmechanismus (718), der mit einem Abschnitt des IV-Schlauchs zum Bereitstellen einer gesteuerten Abgabe eines Fluids aus dem Behälter an den Patienten betriebsfähig ist,
eine Benutzerschnittstelle (710),
einen oder mehrere Prozessoren (712) und
einen Speicher (714), der eine Vielzahl von trainierten Support-Vektor-Maschinen (702) und Anweisungen speichert, die, wenn sie durch den einen oder die mehreren Prozessoren ausgeführt werden, den einen oder die mehreren Prozessoren veranlassen zum:
Empfangen, über den Kraftsensor und während eines Starts einer Infusionssitzung, einer anfänglichen Messung einer physikalischen Kraft, die durch eine Fluidbewegung durch den IV-Schlauch verursacht wird;
Auswählen, basierend auf einem oder mehreren Parametern, die der Infusionssitzung zugeordnet sind, einer trainierten Support-Vektor-Maschine zur Okklusionserkennung aus der Vielzahl der trainierten Support-Vektor-Maschinen;
Erzeugen, während der Infusionssitzung in vorgegebenen Intervallen, eines Merkmalsvektors, der als Eingabe für die ausgewählte trainierte Support-Vektor-Maschine konfiguriert ist, wobei der Merkmalsvektor umfasst:
die anfängliche Messung der durch den Kraftsensor gemessenen physikalischen Kraft,
eine aktuelle Messung der durch den Kraftsensor gemessenen physikalischen Kraft,
eine Langzeitänderung bei der Messung der physikalischen Kraft und
eine Kurzzeitänderung bei der Messung der physikalischen Kraft;
Eingeben, während der Infusionssitzung in den vorgegebenen Intervallen, des Merkmalsvektors in die trainierte Support-Vektor-Maschine, um eine Erkennung eines Vorhandenseins oder eines Nichtvorhandenseins einer Okklusion in dem IV-Schlauch auszugeben; und
wenn das Vorhandensein der Okklusion erkannt wird, Bewirken, dass ein Warnhinweis oder ein Alarm auf der Bedienerschnittstelle angezeigt wird, der auf die Okklusion hinweist.

2. System nach Anspruch 1, wobei der eine oder die mehreren Prozessoren konfiguriert sind, um die Infusionssitzung zu pausieren oder zu beenden, wenn das Vorhandensein der Okklusion erkannt wird.

3. System nach Anspruch 1 oder 2, wobei der Kraftsensor mit dem IV-Schlauch integral ausgebildet oder fluidisch verbunden ist.

4. System nach Anspruch 1, 2 oder 3, wobei das Erzeugen des Merkmalsvektors ferner umfasst:
Empfangen, über den Kraftsensor, der aktuellen Messung der physikalischen Kraft;
Ermitteln, basierend auf einer ersten vorherigen Messung der physikalischen Kraft, der Langzeitänderung bei der Messung der physikalischen Kraft; und
Ermitteln, basierend auf einer zweiten vorherigen Messung der physikalischen Kraft, der Kurzzeitänderung bei der Messung der physikalischen Kraft.

5. System nach Anspruch 1, 2, 3 oder 4, wobei die Anweisungen, wenn sie ausgeführt werden, ferner bewirken, dass der eine oder die mehreren Prozessoren die Support-Vektor-Maschine unter Verwendung eines Trainingsdatensatzes aus einer Vielzahl von Referenzinfusionssitzungen trainieren, denen der eine oder die mehreren Parameter der Infusionssitzung gemeinsam sind.

6. System nach Anspruch 5, wobei das Trainieren der Support-Vektor-Maschine ferner umfasst: Empfangen, für jede der Vielzahl von Referenzinfusionssitzungen:
eines oder mehrerer Referenzmerkmalsvektoren, die einem oder mehreren Zeitpunkten während der jeweiligen Infusionssitzung entsprechen, wobei ein gegebener Referenzmerkmalsvektor umfasst:
eine anfängliche Referenzmessung der physikalischen Kraft zu Beginn der jeweiligen Referenzinfusionssitzung,
eine aktuelle Referenzmessung der physikalischen Kraft zu einem gegebenen Zeitpunkt des einen oder der mehreren Zeitpunkte während der Referenzinfusionssitzung,
eine Referenz-Langzeitänderung bei der Messung der physikalischen Kraft und
eine Referenz-Kurzzeitänderung bei der Messung der physikalischen Kraft und
eine Angabe eines Vorhandenseins oder eines Nichtvorhandenseins einer Okklusion zu dem einen oder den mehreren Zeitpunkten während der jeweiligen Infusionssitzung;
Zuordnen, für jede der Vielzahl von Referenzinfusionssitzungen, des einen oder der mehreren Merkmalsvektoren entweder zu der Angabe des Vorhandenseins der Okklusion oder zu der Angabe des Nichtvorhandenseins der Okklusion;
Ermitteln, basierend auf einer optimierten Marge, einer Entscheidungsgrenze für den Trainingsdatensatz, wobei die Entscheidungsgrenze die Referenzmerkmalsvektoren, die der Angabe des Vorhandenseins der Okklusion zugeordnet sind, von den Referenzmerkmalsvektoren trennt, die der Angabe des Nichtvorhandenseins der Okklusion zugeordnet sind; und
Erzeugen, basierend auf der Entscheidungsgrenze, der trainierten Support-Vektor-Maschine.

7. System nach Anspruch 1, 2 oder 6, wobei die Langzeitänderung bei der Messung der physikalischen Kraft einen Steigungswert einschließt, der durch die aktuelle Messung der physikalischen Kraft über einer Messung der physikalischen Kraft in einer fernen Vergangenheit dargestellt wird.

8. System nach Anspruch 7, wobei die ferne Vergangenheit eine oder mehrere Messungen der physikalischen Kraft einschließt, die ein bis zwei Minuten zuvor aufgezeichnet wurden.

9. System nach Anspruch 1, 2 oder 6, wobei die Langzeitänderung bei der Messung der physikalischen Kraft einen Steigungswert umfasst, der gleich der aktuellen Messung der physikalischen Kraft abzüglich einer Messung der physikalischen Kraft in einer fernen Vergangenheit, dividiert durch eine Anzahl von Messungen, ist, die zwischen der aktuellen Messung der physikalischen Kraft und der Messung der physikalischen Kraft in der fernen Vergangenheit aufgezeichnet wurden.

10. System nach Anspruch 9, wobei die ferne Vergangenheit eine oder mehrere Messungen der physikalischen Kraft einschließt, die ein bis zwei Minuten zuvor aufgezeichnet wurden.

11. System nach Anspruch 1, 2, 4 oder 6, wobei die Kurzzeitänderung bei der Messung der physikalischen Kraft einen Steigungswert einschließt, der durch die aktuelle Messung der physikalischen Kraft über einer Messung der physikalischen Kraft in einer nahen Vergangenheit dargestellt wird.

12. System nach Anspruch 11, wobei die nahe Vergangenheit eine oder mehrere Messungen der physikalischen Kraft einschließt, die 50 Millisekunden bis 100 Millisekunden zuvor aufgezeichnet wurden.

13. System nach Anspruch 1, 2, 4 oder 6, wobei die Kurzzeitänderung bei der Messung der physikalischen Kraft einen Steigungswert einschließt, der gleich der aktuellen Messung der physikalischen Kraft abzüglich einer Messung der physikalischen Kraft in einer nahen Vergangenheit, dividiert durch eine Anzahl von Messungen, ist, die zwischen der aktuellen Messung der physikalischen Kraft und der Messung der physikalischen Kraft in der nahen Vergangenheit aufgezeichnet wurden.

14. System nach Anspruch 13, wobei die nahe Vergangenheit eine oder mehrere Messungen der physikalischen Kraft einschließt, die 50 Millisekunden bis 100 Millisekunden zuvor aufgezeichnet wurden.

15. Infusionspumpenverfahren zum Erkennen einer Okklusion, wobei das Verfahren umfasst:
Empfangen, in einem Prozessor (712) einer Infusionsvorrichtung (700) von einem Kraftsensor (724) und während eines Beginns einer Infusionssitzung, einer anfänglichen Messung einer physikalischen Kraft, die durch Fluidbewegung durch einen IV-Schlauch verursacht wird, der ein erstes Ende, das fluidisch mit einem Patienten gekoppelt ist, und ein zweites Ende aufweist, das fluidisch mit einem Behälter gekoppelt ist;
Erzeugen, über den Prozessor während der Infusionssitzung in vorgegebenen Intervallen, eines Merkmalsvektors, der als Eingabe für eine trainierte Support-Vektor-Maschine (702) konfiguriert ist, wobei der Merkmalsvektor umfasst:
die anfängliche Messung der durch den Kraftsensor gemessenen physikalischen Kraft,
eine aktuelle Messung der durch den Kraftsensor gemessenen physikalischen Kraft,
eine Langzeitänderung bei der Messung der physikalischen Kraft und
eine Kurzzeitänderung bei der Messung der physikalischen Kraft;
Eingeben, über den Prozessor während der Infusionssitzung in den vorgegebenen Intervallen, des Merkmalsvektors in die trainierte Support-Vektor-Maschine, um eine Erkennung eines Vorhandenseins oder eines Nichtvorhandenseins einer Okklusion in dem IV-Schlauch auszugeben; und
wenn das Vorhandensein der Okklusion erkannt wird, Pausieren oder Beenden der Infusionssitzung über den Prozessor.

## Revendications

1. Système de perfusion permettant de détecter une occlusion, le système comprenant :
un capteur de force (724) couplé à une tubulure IV pour mesurer une force physique provoquée par un fluide au sein de la tubulure IV, la tubulure IV ayant une première extrémité qui est raccordée fluidiquement à un patient et une seconde extrémité qui est raccordée fluidiquement à un récipient ; et
un dispositif de perfusion (700) couplé en communication au capteur de force, le dispositif de perfusion comportant :
un mécanisme de pompage (718) pouvant fonctionner avec une partie de la tubulure IV pour fournir une administration régulée d'un fluide, du récipient au patient,
une interface utilisateur (710),
un ou plusieurs processeurs (712), et
une mémoire (714) stockant une pluralité de machines à vecteurs de support entraînées (702) et des instructions qui, lorsqu'elles sont exécutées par le ou les processeurs, amènent le ou les processeurs à :
recevoir, par le biais du capteur de force et lors d'un début d'une session de perfusion, une mesure initiale d'une force physique provoquée par un mouvement de fluide à travers la tubulure IV ;
sélectionner, sur la base d'un ou plusieurs paramètres associés à la session de perfusion, une machine à vecteurs de support entraînée pour une détection d'occlusion parmi la pluralité des machines à vecteurs de support entraînées ;
générer, pendant la session de perfusion à des intervalles prédéterminés, un vecteur de caractéristiques configuré en tant qu'entrée pour la machine à vecteurs de support entraînée sélectionnée, le vecteur de caractéristiques comprenant :
la mesure initiale de la force physique mesurée par le capteur de force,
une mesure actuelle de la force physique mesurée par le capteur de force,
un changement à long terme de la mesure de la force physique, et
un changement à court terme de la mesure de la force physique ;
entrer, pendant la session de perfusion aux intervalles prédéterminés, le vecteur de caractéristiques dans la machine à vecteurs de support entraînée afin de délivrer une détection d'une présence ou d'une absence d'une occlusion dans la tubulure IV ; et
lorsque la présence de l'occlusion est détectée, provoquer l'affichage d'une alerte ou d'une alarme sur l'interface utilisateur qui indique l'occlusion.

2. Système selon la revendication 1, dans lequel le ou les processeurs sont configurés pour suspendre ou terminer la session de perfusion lorsque la présence de l'occlusion est détectée.

3. Système selon les revendications 1 ou 2, dans lequel le capteur de force est formé d'un seul tenant ou raccordé fluidiquement à la tubulure IV.

4. Système selon les revendications 1, 2, ou 3, dans lequel la génération du vecteur de caractéristiques comprend en outre :
la réception, par le biais du capteur de force, de la mesure actuelle de la force physique ;
la détermination, sur la base d'une première mesure antérieure de la force physique, du changement à long terme de la mesure de la force physique ; et
la détermination, sur la base d'une seconde mesure antérieure de la force physique, du changement à court terme de la mesure de la force physique.

5. Système selon les revendications 1, 2, 3, ou 4, dans lequel les instructions, lorsqu'elles sont exécutées, amènent en outre le ou les processeurs à entraîner la machine à vecteurs de support à l'aide d'un ensemble de données d'entraînement provenant d'une pluralité de sessions de perfusion de référence partageant le ou les paramètres de la session de perfusion.

6. Système selon la revendication 5, dans lequel l'entraînement de la machine à vecteurs de support comprend en outre : la réception, pour chacune de la pluralité de sessions de perfusion de référence, de :
un ou plusieurs vecteurs de caractéristiques de référence correspondant à un ou plusieurs instants pendant la session de perfusion respective, dans lequel un vecteur de caractéristiques de référence donné comprend :
une mesure initiale de référence de la force physique au début de la session de perfusion de référence respective,
une mesure de courant de référence de la force physique à un instant donné du ou des instants pendant la session de perfusion de référence,
un changement à long terme de référence de la mesure de la force physique, et
un changement à court terme de référence de la mesure de la force physique, et
une indication d'une présence ou d'une absence d'une occlusion à l'instant ou aux instants pendant la session de perfusion respective ;
l'association, pour chacune de la pluralité de sessions de perfusion de référence, du ou des vecteurs de caractéristiques soit à l'indication de la présence de l'occlusion, soit à l'indication de l'absence de l'occlusion ;
la détermination, sur la base d'une marge optimisée, d'une frontière de décision pour l'ensemble de données d'entraînement, dans lequel la frontière de décision sépare les vecteurs de caractéristiques de référence associés à l'indication de la présence de l'occlusion des vecteurs de caractéristiques de référence associés à l'indication de l'absence de l'occlusion ; et
la génération, sur la base de la frontière de décision, de la machine à vecteurs de support entraînée.

7. Système selon les revendications 1, 2, ou 6, dans lequel le changement à long terme de la mesure de la force physique comporte une valeur de pente représentée par la mesure actuelle de la force physique sur une mesure de la force physique dans un passé lointain.

8. Système selon la revendication 7, dans lequel le passé lointain comporte une ou plusieurs mesures de la force physique enregistrées une à deux minutes auparavant.

9. Système selon les revendications 1, 2, ou 6, dans lequel le changement à long terme de la mesure de la force physique comporte une valeur de pente qui est égale à la mesure actuelle de la force physique moins une mesure de la force physique dans un passé lointain, divisée par un nombre de mesures enregistrées entre la mesure actuelle de la force physique et la mesure de la force physique dans le passé lointain.

10. Système selon la revendication 9, dans lequel le passé lointain comporte une ou plusieurs mesures de la force physique enregistrées une à deux minutes auparavant.

11. Système selon les revendications 1, 2, 4, ou 6, dans lequel le changement à court terme de la mesure de la force physique comporte une valeur de pente représentée par la mesure actuelle de la force physique sur une mesure de la force physique dans un passé proche.

12. Système selon la revendication 11, dans lequel le passé proche comporte une ou plusieurs mesures de la force physique enregistrées de 50 millisecondes à 100 millisecondes auparavant.

13. Système selon les revendications 1, 2, 4, ou 6, dans lequel le changement à court terme de la mesure de la force physique comporte une valeur de pente qui est égale à la mesure actuelle de la force physique moins une mesure de la force physique dans un passé proche, divisée par un nombre de mesures enregistrées entre la mesure actuelle de la force physique et la mesure de la force physique dans le passé proche.

14. Système selon la revendication 13, dans lequel le passé proche comporte une ou plusieurs mesures de la force physique enregistrées de 50 millisecondes à 100 millisecondes auparavant.

15. Procédé de pompe à perfusion permettant de détecter une occlusion, le procédé comprenant :
la réception, dans un processeur (712) d'un dispositif de perfusion (700) en provenance d'un capteur de force (724) et au cours d'un début d'une session de perfusion, d'une mesure initiale d'une force physique provoquée par un mouvement de fluide à travers une tubulure IV ayant une première extrémité qui est raccordée fluidiquement à un patient et une seconde extrémité qui est raccordée fluidiquement à un récipient ;
la génération, par le biais du processeur pendant la session de perfusion à des intervalles prédéterminés, d'un vecteur de caractéristiques configuré en tant qu'entrée pour une machine à vecteurs de support entraînée (702), le vecteur de caractéristiques comprenant :
la mesure initiale de la force physique mesurée par le capteur de force,
une mesure actuelle de la force physique mesurée par le capteur de force,
un changement à long terme de la mesure de la force physique, et
un changement à court terme de la mesure de la force physique ;
l'entrée, par le biais du processeur pendant la session de perfusion aux intervalles prédéterminés, du vecteur de caractéristiques dans la machine à vecteurs de support entraînée afin de délivrer une détection d'une présence ou d'une absence d'une occlusion dans la tubulure IV ; et
lorsque la présence de l'occlusion est détectée, le fait de suspendre ou de terminer la session de perfusion par le biais du processeur.
